# EUROPEAN PATENT SPECIFICATION

(11) **EP 3 520 751 B1**
(45) Date of publication and mention of the grant of the patent: **06.09.2023**
(21) Application number: 16917756.5
(22) Date of filing: 30.09.2016
(51) Int. Cl.: A61F 13/15

(54) **METHOD FOR MANUFACTURING ABSORBENT ARTICLE**
VERFAHREN ZUR HERSTELLUNG EINES ABSORBIERENDEN ARTIKELS
PROCÉDÉ DE FABRICATION D'UN ARTICLE ABSORBANT

(43) Date of publication of application: 07.08.2019
(73) Proprietor: Unicharm Corporation, Ehime 799-0111 (JP)
(72) Inventor: TAKEUCHI, Kenji, Kanonji-shi Kagawa 769-1602 (JP); YOKOYAMA, Takumi, Kanonji-shi Kagawa 769-1602 (JP)
(74) Representative: Staeger & Sperling Partnerschaftsgesellschaft mbB
(86) International application number: PCT/JP2016/079141
(87) International publication number: WO 2018/061206

(56) References cited:
- WO-A1-2015/025760
- JP-A- 2008 125 627
- JP-A- 2013 081 769
- JP-A- 2014 050 749
- JP-B1- 5 779 737

## Description

### TECHNICAL FIELD

The present invention relates to a method for manufacturing an absorbent article in which a structure of a front edge of an absorbent main body containing an absorber is different from a structure of a rear edge of the absorbent main body.

### BACKGROUND ART

Patent Literature 1 discloses a method for manufacturing an absorbent article in which a structure of a front edge of an absorbent main body is different from a structure of a rear edge of the absorbent main body. In the method for manufacturing the absorbent article of Patent Literature 1, a main body continuum in which absorbent main bodies are continuous in a longitudinal direction is formed, and the individual absorbent main bodies are obtained by cutting the main body continuum. In the main body continuum, front edges of absorbent main bodies are adjacent to each other, and rear edges of absorbent main bodies are adjacent to each other (for example, Fig. 7 of Patent Literature 1).

In the method for manufacturing the absorbent article of Patent Literature 1, the individual absorbent main bodies are rotated in a certain direction (for example, clockwise in Fig. 7 of Patent Literature 1), and the absorbent main bodies are disposed on a waistline continuum in which waistline members are continuous. In a state in which the absorbent main bodies are disposed on the waistline continuum, the front edges of the absorbent main bodies and the rear edges of the absorbent main bodies are alternately arranged along a conveyance direction (for example, Fig. 4 of Patent Literature 1). In the waistline continuum, a region disposed on a rear waistline region side and a region disposed on a front waistline region side are alternately arranged along the conveyance direction.

Patent Literature 2 discloses a manufacturing method in which a front edge of an absorbent main body and a rear edge of an absorbent main body are alternately disposed on a waistline continuum along a conveyance direction to manufacture an absorbent article. In the waistline continuum of Patent Literature 2, openings constituting leg surrounding openings are formed at intervals in the conveyance direction. One opening constitutes a leg surrounding opening on a front waistline region side and a leg surrounding opening on a rear waistline region side adjacent to each other in the conveyance direction (for example, Fig. 6 of Patent Literature 2).

### CITATION LIST

### PATENT LITERATURE

Patent Literature 1: JP 2011-103943 A
Patent Literature 2: JP 2000-237233 A

### SUMMARY OF INVENTION

In the waistline continuum of Patent Literature 1 and Patent Literature 2, the region disposed on the rear waistline region side and the region disposed on the front waistline region side are alternately arranged along the conveyance direction, and a structure of the region disposed on the front waistline region side is the same as a structure of the region disposed on the rear waistline region side. Specifically, when waistline elastic members continuous in the conveyance direction are provided, extension stress of the absorbent article on the front waistline region side is the same as extension stress on the rear waistline region side. Since extension stress of the absorbent article on the front waistline region side is the same as extension stress on the rear waistline region side, the absorbent article fails to fit both a ventral portion and a gluteal portion in some cases.

In the leg surrounding opening of the absorbent article of Patent Literature 2, a shape on the front waistline region side is different from a shape on the rear waistline region side. In addition, in the waistline continuum, the region disposed on the front waistline region side and the region disposed on the rear waistline region side are alternately arranged along the conveyance direction. Therefore, two opening patterns are formed in the waistline continuum, and opening patterns are different every other pattern. One pitch of an opening pattern corresponds to a length of two absorbent articles. In this way, when a pitch of a processing pattern such as an opening corresponds to a length of two absorbent articles, a production facility increases in size as compared to an apparatus in which a pitch of a processing pattern corresponds to a length of one absorbent article. When the production facility increases in size, exchange equipment at the time of changing a processing pattern becomes larger.

The invention has been made in view of the above-mentioned problem, and an object of the invention is to provide a method for manufacturing an absorbent article capable of manufacturing an absorbent article in which a structure of a front edge of an absorbent main body containing an absorber is different from a structure of a rear edge of the absorbent main body and extension stress of a front waistline region is different from extension stress of a rear waistline region while suppressing an increase in size of a production facility.

A method for manufacturing an absorbent article according to a present invention is a method for manufacturing an absorbent article according to claim 1 including an absorbent main body that contains an absorber and has a longitudinal direction and a width direction orthogonal to the longitudinal direction, and a waistline member that includes a waistline elastic member extending in the width direction and that constitutes in a front waistline region and a rear waistline region, a structure of a front edge of the absorbent main body being different from a structure of a rear edge of the absorbent main body, and an extension stress of the front waistline region being different from an extension stress of the rear waistline region. The method for manufacturing the absorbent article comprises: a main body conveyance step of conveying a main body continuum in which the absorbent main bodies are continuous in the longitudinal direction, the front edges of the absorbent main bodies are adjacent to each other, and the rear edges of the absorbent main bodies are adjacent to each other; a cutting step of cutting the main body continuum along the width direction to obtain the individual absorbent main bodies; a waistline conveyance step of conveying a waistline continuum in which regions constituting the front waistline regions are continuous in a conveyance direction, and regions constituting the rear waistline regions are continuous in the conveyance direction; and a main body arrangement step of rotating the absorbent main bodies and disposing the absorbent main bodies on the waistline continuum. In the main body arrangement step, after absorbent main bodies adjacent to each other in the conveyance direction are rotated in opposite directions, the front edges of the absorbent main bodies are disposed in a region of the waistline continuum on a side of the front waistline regions, and the rear edges of the absorbent main bodies are disposed in a region of the waistline continuum on a side of the rear waistline regions.

### BRIEF DESCRIPTION OF DRAWINGS

Fig. 1 is a plan view of an absorbent article in an embodiment.
Fig. 2 is a schematic view illustrating an outline of a main body conveyance step and an elastic member arrangement step in a method for manufacturing the absorbent article.
Fig. 3 is a schematic view illustrating an outline of a cutting step and a main body arrangement step of the method for manufacturing the absorbent article.
Fig. 4 is a schematic view illustrating an outline of a waistline conveyance step, a main body arrangement step, and an extension and contraction suppression processing step of the method for manufacturing the absorbent article.
Fig. 5 is a diagram for description of the main body arrangement step.

### DESCRIPTION OF EMBODIMENTS

At least the following matters will become apparent from the description of this specification and accompanying drawings.

A method for manufacturing an absorbent article,
the absorbent article including an absorbent main body that contains an absorber and has a longitudinal direction and a width direction orthogonal to the longitudinal direction, and a waistline member that includes a waistline elastic member extending in the width direction and that constitutes in a front waistline region and a rear waistline region,
a structure of a front edge of the absorbent main body being different from a structure of a rear edge of the absorbent main body, and
an extension stress of the front waistline region being different from an extension stress of the rear waistline region,
the method comprising:
   a main body conveyance step of conveying a main body continuum in which the absorbent main bodies are continuous in the longitudinal direction, the front edges of the absorbent main bodies are adjacent to each other, and the rear edges of the absorbent main bodies are adjacent to each other;
   a cutting step of cutting the main body continuum along the width direction to obtain the individual absorbent main bodies;
   a waistline conveyance step of conveying a waistline continuum in which regions constituting the front waistline regions are continuous in a conveyance direction, and regions constituting the rear waistline regions are continuous in the conveyance direction; and
   a main body arrangement step of rotating the absorbent main bodies and disposing the absorbent main bodies on the waistline continuum,
   wherein in the main body arrangement step, after absorbent main bodies adjacent to each other in the conveyance direction are rotated in opposite directions, the front edges of the absorbent main bodies are disposed in a region of the waistline continuum on a side of the front waistline regions, and the rear edges of the absorbent main bodies are disposed in a region of the waistline continuum on a side of the rear waistline regions.

Absorbent main bodies are continuous in a longitudinal direction, front edges of absorbent main bodies are adjacent to each other, and rear edges of absorbent main bodies are adjacent to each other. From such a main body continuum, it is possible to obtain an absorbent main body in which a structure of a front edge of the absorbent main body is different from a structure of a rear edge of the absorbent main body. In the front edge of the absorbent main body and the rear edge of the absorbent main body, the number, a material, and a width direction length of sheets, a position of an elastic member in the width direction, etc. can easily be made different.

To rotate the absorbent main bodies obtained by cutting the main body continuum alternately in opposite directions, the front edges of the absorbent main bodies may be disposed in a region on a front waistline region side of the waistline continuum, and the rear edges of the absorbent main bodies may be disposed in a region on a rear waistline region side thereof by rotation at an angle larger than 0 degree and smaller than 180 degrees. Since the front edges of the absorbent main bodies are aligned with each other and the rear edges of the absorbent main bodies are aligned with each other on the waistline continuum, regions continuous in a conveyance direction in the waistline continuum constitute only in front waistline regions or rear waistline regions. Therefore, extension magnification or the number of waistline elastic members may be made different between the front waistline region and the rear waistline region, and it is possible to manufacture an absorbent article in which extension stress of the front waistline region is different from extension stress of the rear waistline region. It is possible to manufacture an absorbent article capable of realizing a fitting fit to both a ventral portion and a gluteal portion. In addition, since absorbent articles are manufactured in a state in which the front waistline regions are continuous and the rear waistline regions are continuous in the conveyance direction, a pitch of a processing pattern corresponds to a length of one absorbent article, and an increase in size of a facility can be suppressed. Since it is possible to suppress an increase in size of a production facility, exchange equipment at the time of changing the processing pattern becomes small, and replacement work can be easily performed.

In such a method for manufacturing the absorbent article, it is preferable that in the main body arrangement step the absorbent main bodies adjacent to each other in the conveyance direction are rotated by 90 degrees in opposite directions.

When the absorbent main bodies are alternately rotated by 90 degrees, rotation angles of all the absorbent main bodies are the same, and the absorbent main bodies can be arranged in a certain direction. Since the absorbent main bodies are rotated by 90 degrees at a time, rotation can be realized by a substantially common mechanism even in a reverse rotation. Addition to the mechanism due to acceleration etc. accompanying rotation corresponds to the same degree, and can be easily designed while suppressing defect of the mechanism.

In such a method for manufacturing the absorbent article,
it is preferable that the main body arrangement step aligns positions of the front edges of the absorbent main bodies in an orthogonal direction orthogonal to the conveyance direction.

Since the positions of the front edges of the absorbent main bodies in the orthogonal direction coincide with each other, it is possible to continuously manufacture absorbent articles in which positions of the absorbent main bodies in the orthogonal direction are uniform. In addition, by causing positions of turning centers of a turning mechanism that turns the absorbent main bodies to coincide with each other in the orthogonal direction, it is possible to suppress complication of a design of the turning mechanism when compared to a mechanism in which positions of turning centers are shifted in the orthogonal direction.

In such a method for manufacturing the absorbent article, it is
according to the invention that a length of the rear edge of the absorbent main body in the width direction is longer than a length of the front edge of the absorbent main body in the width direction.

Since the main body continuum in which the front edges of the absorbent main bodies are adjacent to each other and the rear edges of the absorbent main bodies are adjacent to each other is formed, it is possible to manufacture the absorbent main body in which the length of front edge of the absorbent main body in the width direction is different from the length of rear edge of the absorbent main body in the width direction. In the absorbent article configured in this way, a length in the width direction on the rear waistline region side is relatively long, and the gluteal portion can be more appropriately covered.

In such a method for manufacturing the absorbent article,
it is preferable that the method further comprises an elastic member arrangement step of providing a main body elastic member that extends and contracts in the longitudinal direction on the main body continuum before the cutting step,
wherein the elastic member arrangement step makes a position of a front edge of the main body elastic member in the width direction different from a position of a rear edge of the main body elastic member in the width direction.

Since the main body continuum in which the front edges of the absorbent main bodies are adjacent to each other and the rear edges of the absorbent main bodies are adjacent to each other is formed, it is possible to manufacture the absorbent main body in which the positions of the front edges of the main body elastic members in the width direction are different from the positions of the rear edges of the main body elastic members in the width direction. In addition, when the rear edge of the main body elastic member is disposed on the outside of the front edge of the main body elastic member in the width direction, the main body elastic member on the side of the rear waistline region may be provided on a relatively outer side in the width direction, so that it is possible to obtain the absorbent article more appropriately covering the gluteal portion.

In such a method for manufacturing the absorbent article,
it is preferable that the main body arrangement step disposes the absorbent main body on the waistline continuum such that at least a part of the main body elastic member overlaps the waistline elastic member.

According to the absorbent article configured as described above, the main body elastic members are pulled up by the waistline elastic members at the time of wearing, and the absorbent main body fits more to a body of the wearer.

In such a method for manufacturing the absorbent article,
it is preferable that the waistline conveyance step separately conveys a front waistline continuum and a rear waistline continuum constituting the waistline continuum, and the main body arrangement step disposes the absorbent main body so that the absorbent main body straddles the front waistline continuum and the rear waistline continuum.

By separately providing the front waistline continuum and the rear waistline continuum, it is possible to form the front waistline region and the rear waistline region using raw materials such as different materials. Therefore, it is possible to manufacture the absorbent article realizing a texture and a shape more suitable for the ventral portion and the gluteal portion.

In such a method for manufacturing the absorbent article,
it is preferable that a structure of the front waistline continuum is different from a structure of the rear waistline continuum.

By forming the front waistline region and the rear waistline region having different structures, it is possible to manufacture the absorbent article realizing a texture and a shape more suitable for the ventral portion and the gluteal portion.

In such a method for manufacturing the absorbent article,
it is preferable that the waistline elastic member is disposed in each of the front waistline region and the rear waistline region,
the method further comprises an extension and contraction suppression processing step of suppressing a stretching force of the waistline elastic member, and a processing pattern of the front waistline region is different from a processing pattern of the rear waistline region in the extension and contraction suppression processing step.

Regions continuous in the conveyance direction in the waistline continuum constitutes only in front waistline regions or rear waistline regions. Therefore, the pitch of the processing pattern corresponds to the length of one absorbent article, and it is possible to make a processing pattern for suppressing extension and contraction of the waistline elastic member different between the front waistline region and the rear waistline region while suppressing an increase in size of the facility. According to the absorbent article manufactured as described above, an extension and contraction mode of the front waistline region is different from an extension and contraction mode of the rear waistline region, and it is possible to realize a fitting more suitable for each of the ventral portion and the gluteal portion.

### === With regard to absorbent article according to present embodiment ===

Hereinafter, a method for manufacturing the absorbent article according to the embodiment will be described with reference to the drawings. In the following description of the drawings, the same or similar reference symbols are attached to the same or similar parts. However, it should be noted that the drawings are schematic and ratios of dimensions, etc. are different from actual ones. Therefore, specific dimensions, etc. should be determined with reference to the following description. In addition, drawings may include portions having different dimensional relationships or ratios.

Examples of the absorbent article manufactured by the method for manufacturing the absorbent article may include a disposable diaper, a sanitary napkin, and an absorbent pad. In the present embodiment, a manufacturing method for manufacturing a disposable diaper will be described.

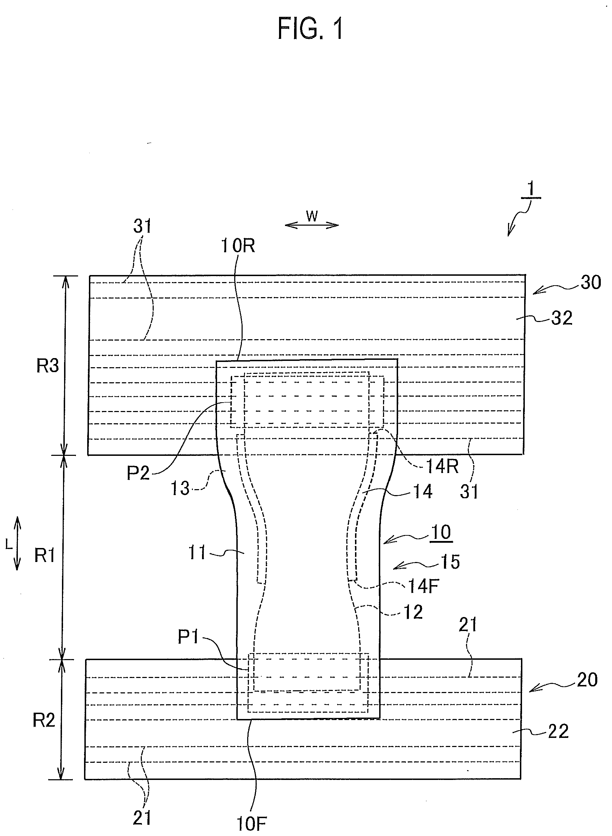

An absorbent article 1 is a pants-type disposable diaper, and Fig. 1 is a plan view of the absorbent article 1 in an unfolded state. The absorbent article 1 includes a front waistline region R2 disposed on a ventral portion side of a wearer, a rear waistline region R3 disposed on a gluteal portion side of the wearer, and a crotch region R1 disposed between the front waistline region R2 and the rear waistline region R3 and disposed in a crotch of the wearer. The front waistline region R2 is a region where a front waistline member 20 is disposed and the rear waistline region R3 is a region where a rear waistline member 30 is disposed.

The absorbent article 1 has an absorbent main body 10 and a waistline member. The absorbent article 1 has a longitudinal direction L and a width direction W orthogonal to the longitudinal direction L. The absorbent main body 10 has at least a front sheet 11, an absorber 12, a rear sheet 13, and main body elastic members 14. The front sheet 11 is disposed on a skin contact surface side of the absorber 12 and has liquid permeability. The rear sheet 13 is disposed on a non-skin contact surface side of the absorber 12 and has liquid impermeability. The absorber 12 is disposed between the front sheet 11 and the rear sheet 13. The absorber 12 has an hourglass shape in plan view and a front-rear asymmetric shape. The main body elastic members 14 are stretchable in the longitudinal direction. The main body elastic members 14 are fixed in a state of being stretched in the longitudinal direction between the front sheet 11 and the rear sheet 13 on an outer side of the absorber 12 in the width direction.

A structure of a front edge 10F of the absorbent main body 10 and a structure of a rear edge 10R of the absorbent main body 10 are different from each other. Here, a structure of the absorbent main body includes a length of the absorbent main body in the width direction, the number of constituent members constituting the absorbent main body, materials of the constituent members, a position of the elastic member, etc. At least one of the shape and the material is different between the front edge 10F of the absorbent main body 10 and the rear edge 10R of the absorbent main body 10. The absorbent main body 10 has a front-rear asymmetric shape. A shape of the ventral portion and a shape of the gluteal portion of the wearer are different from each other. According to the absorbent article in which the structure of the front edge 10F of the absorbent main body 10 and the structure of the rear edge 10R of the absorbent main body 10 are different from each other, the absorbent article may be more appropriately fit to each of the ventral portion and the gluteal portion of the wearer. A length of the rear edge 10R of the absorbent main body 10 in the width direction W may be longer than a length of the front edge 10F of the absorbent main body 10 in the width direction W In the absorbent article configured in this way, a length in the width direction on the rear waistline region side is relatively long, and the gluteal portion can be more appropriately covered.

The waistline member has at least the front waistline member 20 constituting the front waistline region R2, the rear waistline member 30 constituting the rear waistline region R3, and a waistline elastic member extending in the width direction. The waistline elastic member is stretchable in the width direction W A front waistline elastic member 21 disposed in the front waistline region R2 and a rear waistline elastic member 31 disposed in the rear waistline region R3 are included. The front waistline member 20 has at least two sheet materials 22, and the rear waistline member 30 has at least two sheet materials. The front waistline elastic member 21 is fixed in a state of being stretched in the width direction between the sheet materials 22 of the front waistline member 20. The rear waistline elastic member 31 is fixed in a state of being stretched in the width direction between the sheet materials 32 of the rear waistline member 30.

Extension stress of the front waistline region R2 is different from extension stress of the rear waistline region R3. At least one of the number of elastic members, extension magnification at the time of being fixed to the sheet, and an interval between elastic members is different between the front waistline elastic member 21 and the rear waistline elastic member 31. Here, extension stress is stress at the time of being stretched. As extension stress increases, stretch becomes more difficult, and fitting to a body increases. For example, when extension stress of the rear waistline region R3 is set to be higher than extension stress of the front waistline region, it is possible to enhance adhesion to the gluteal portion without excessively increasing the adhesion to the ventral portion of the wearer.

Extension stress can be measured using a tensile testing machine manufactured by Shimadzu Corporation. For measurement, samples are cut from the front waistline region R2 and the rear waistline region R3, respectively. First, in a state in which one end of a sample is fixed to an upper chuck of a tensile test, and the other end of the sample is fixed to a lower chuck, the ends are spaced 180 mm (a size of a gripping part of the chuck is about 15 mm) apart. Subsequently, the lower chuck is moved at a speed of 100 mm/min to separate the chucks until a distance between the chucks becomes 460 mm, movement is inverted, and the chucks are brought close to each other until the distance between the chucks becomes 180 mm. Thereafter, movement is inverted again, and a measured value at the time when the chucks are separated until the distance between the chucks becomes 460 mm may be set to extension stress (N).

A front stretch suppressed region P1 in which a stretching force of the front waistline elastic member 21 is suppressed is provided in the front waistline region R2, and a rear stretch suppressed region P2 in which a stretching force of the rear waistline elastic member 31 is suppressed is provided in the rear waistline region R3. Shapes of the front stretch suppressed region P1 and the rear stretch suppressed region P2 are different from each other. The shape of the stretch suppressed region includes an area a position, and a length. Since the shapes of the front stretch suppressed region P1 and the rear stretch suppressed region P2 are different from each other, it is possible to realize a fitting fit to both the ventral portion and the gluteal portion.

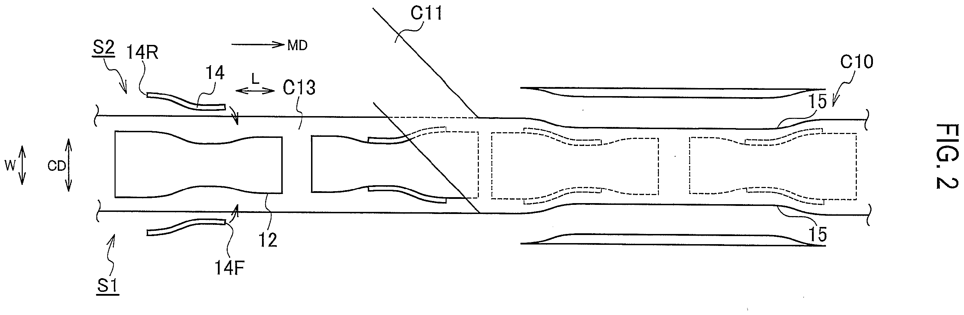

Next, a description will be given of a method for manufacturing the absorbent article of configuring such an absorbent article with reference to Fig. 2 to Fig. 5. The method for manufacturing the absorbent article includes a main body conveyance step S1, an elastic member arrangement step S2, a cutting step S3, a waistline conveyance step S4, a main body arrangement step S5, and an extension and contraction suppression processing step S6.

In the main body conveyance step S1, a main body continuum C10 is formed, and the main body continuum C10 is conveyed. In the main body continuum, absorbent main bodies 10 are continuous in the longitudinal direction L, front edges 10F of the absorbent main bodies 10 are adjacent to each other, and rear edges 10R thereof are adjacent to each other. The longitudinal direction L of the absorbent main body 10 extends along the conveyance direction MD of the main body conveyance step S1, and the width direction W of the absorbent main body 10 extends along the orthogonal direction CD orthogonal to the conveyance direction MD. The main body conveyance step may not have a process of forming the main body continuum, and may only correspond to a process of conveying the main body continuum.

As illustrated in Fig. 2, in the main body conveyance step S1, a rear surface continuum C13 in which rear sheets 13 are continuous in the longitudinal direction L is conveyed, and absorbers 12 are disposed on the rear surface continuum C13 at intervals in the longitudinal direction L. In a configuration including the main body elastic members 14, as the elastic member arrangement step S2, main body elastic members 14 are fixed on the rear surface continuum C13 in a state of being stretched in the longitudinal direction L. Subsequently, as the main body conveyance step S1, a front surface continuum C11 in which front sheets 11 are continuous in the longitudinal direction L is conveyed, and the front surface continuum C11 is disposed on a skin contact surface side of the rear surface continuum C13 and the absorber 12. In the main body conveyance step S1, both sides of the front surface continuum C11 and the rear surface continuum C13 in the width direction are cut along a leg surrounding opening 15 of the absorbent article. In this way, it is possible to obtain the main body continuum C10 in which the absorbent main bodies 10 are continuous in the longitudinal direction L, the front edges 10F of the absorbent main bodies 10 are adjacent to each other, and the rear edges 10R thereof are adjacent to each other.

In the elastic member arrangement step S2, a position of a front edge 14F of each of the main body elastic members 14 in the width direction W may be different from a position of a rear edge 14R of each of the main body elastic members 14 in the width direction. A position of the main body elastic member 14 in the width direction W refers to a position of an outer side edge of the main body elastic member, and corresponds to an outer side edge at a position having a maximum width in a curved main body elastic member. When the position of the front edge 14F of the main body elastic member 14 in the width direction W is different from the position of the rear edge 14R of the main body elastic member 14 in the width direction W, the main body elastic member 14 can be disposed at a more appropriate position with respect to each of the ventral portion and the gluteal portion. For example, when the rear edge 14R of the main body elastic member 14 is disposed on the outside of the front edge 14F of the main body elastic member 14 in the width direction W, the main body elastic member 14 on the side of the rear waistline region R3 is provided on a relatively outer side in the width direction W, so that it is possible to obtain the absorbent article 1 more appropriately covering the gluteal portion. The elastic member arrangement step S2 is an arbitrary step and may be carried out as necessary. Further, the elastic member arrangement step S2 may be performed before the cutting step S3.

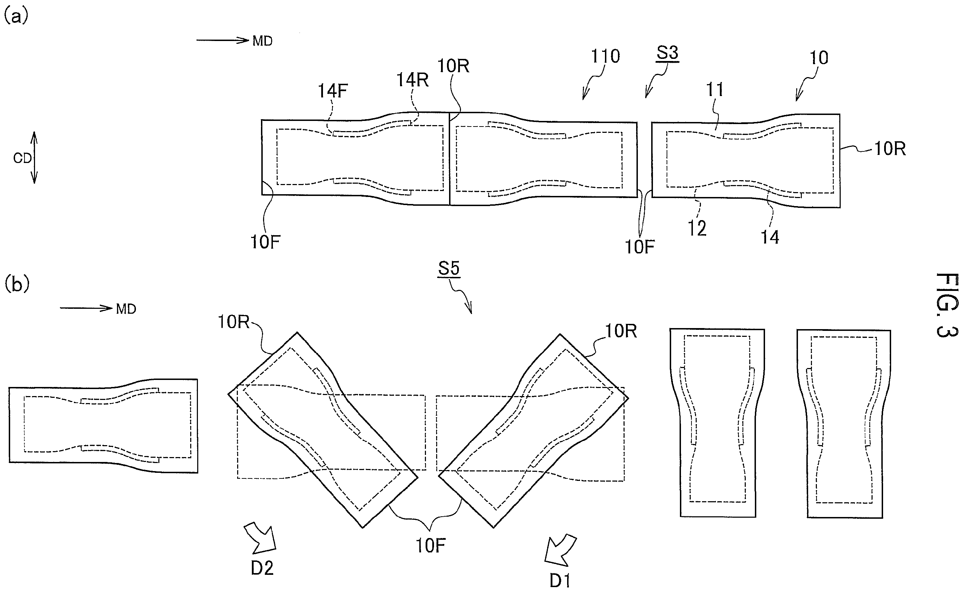

Fig. 3(a) is a diagram schematically illustrating the cutting step S3, and Fig. 3(b) is a diagram schematically illustrating a part (a process of rotating the absorbent main body) of the main body arrangement step S5. Fig. 3(b) corresponds to a downstream side of Fig. 3(a) in the conveyance direction MD. As illustrated in Fig. 3(a), in the cutting step S3, the main body continuum C10 is cut along the width direction W to obtain the individual absorbent main bodies 10. In a state in which the main body continuum C10 is cut along the width direction W, the front edges 10F of the absorbent main bodies 10 adjacent to each other in the conveyance direction MD face each other, and the rear edges 10R of the absorbent main bodies 10 adjacent to each other in the conveyance direction MD face each other. For example, in the main body continuum C10 in which the front edges 10F and the rear edges 10R of the absorbent main bodies 10 are adjacent to each other, the structure of the front edge 10F of the absorbent main body 10 is the same as the structure of the rear edge of the absorbent main body 10. However, according to the main body continuum C10 of the present embodiment, it is possible to easily obtain the absorbent main body 10 in which the structure of the front edge 10F of the absorbent main body 10 is different from the structure of the rear edge 10R of the absorbent main body 10. In the front edge 10F of the absorbent main body 10 and the rear edge 10R of the absorbent main body 10, the number, a material, and a width direction length of sheets, a position of an elastic member in the width direction, etc. can easily be made different, and it is possible to obtain the absorbent article that can fit more to a shape of the body.

In the waistline conveyance step S4, the waistline continuum is formed, and the waistline continuum is conveyed. The waistline continuum has a front waistline continuum C20 in which regions constituting front waistline regions R2 are continuous in the width direction W, and a rear waistline continuum C30 in which regions constituting rear waistline regions R3 are continuous in the width direction W The width direction W of the front waistline region R2 and the width direction W of the rear waistline region extend along the conveyance direction MD of the waistline conveyance step S4. The front waistline continuum C20 and the rear waistline continuum C30 are spaced apart in the orthogonal direction CD. The waistline conveyance step may not have a process of forming the waistline continuum, and may only correspond to a process of conveying the waistline continuum.

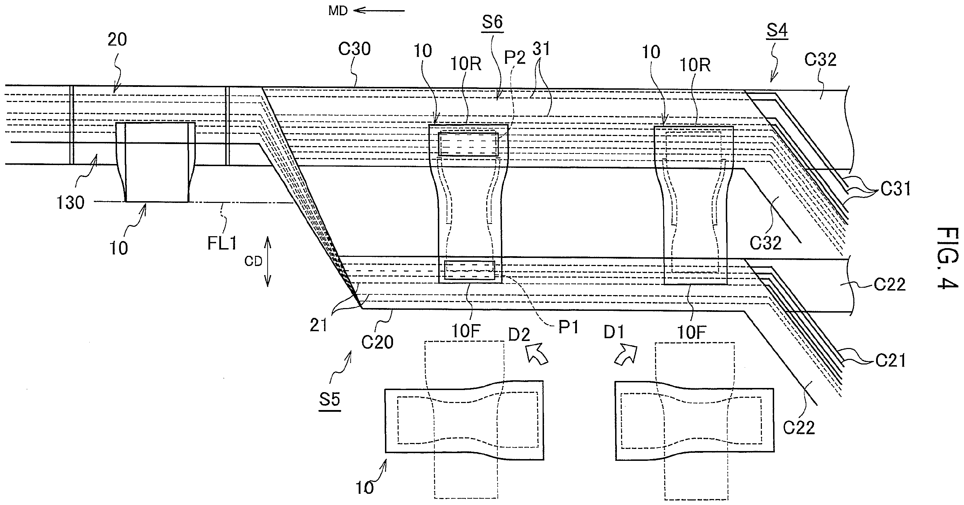

As illustrated in Fig 4, in the waistline conveyance step S4, a continuum C21 of front waistline elastic members 21 extending in the width direction W is continuously conveyed and fixed between continuums C22 of sheet materials 22 constituting front waistline members 20, and a continuum C31 of rear waistline elastic members 31 extending in the width direction W is continuously conveyed and fixed between continuums C32 of sheet materials 32 constituting rear waistline members 30.

In this way, it is possible to obtain the rear waistline continuum C30 in which the rear waistline regions R3 are continuous in the width direction W and the front waistline continuum C20 in which the front waistline regions R2 are continuous in the width direction W Regions continuous in the conveyance direction MD in the waistline continuum constitute only in the front waistline regions R2 or the rear waistline regions R3. Therefore, extension magnification or the number of waistline elastic members may be made different between the front waistline region R2 and the rear waistline regions R3, and it is possible to manufacture the absorbent article 1 in which extension stress of the front waistline region R2 is different from extension stress of the rear waistline regions R3. In addition, since the absorbent article 1 is manufactured in a state in which the front waistline regions R2 are continuous and the rear waistline regions R3 are continuous in the conveyance direction MD, a pitch of a processing pattern corresponds to a length of one absorbent article, and an increase in size of a facility can be suppressed.

In the waistline conveyance step S4, the front waistline continuum C20 and the rear waistline continuum C30 may be separately conveyed. When the front waistline continuum C20 and the rear waistline continuum C30 are separately provided, a structure of the front waistline continuum C20 may be easily made different from a structure of the rear waistline continuum C30. The structure of the waistline continuum includes a shape (a length in the orthogonal direction), the number or a material of constituent members constituting the waistline continuum, a position of an elastic member, etc. It is possible to easily obtain the absorbent article 1 in which a structure of the front waistline region R2 is different from a structure of the rear waistline region R3, and it is possible to realize a texture and a shape more suitable for the ventral portion and the gluteal portion. The front waistline continuum C20 and the rear waistline continuum C30 of the present embodiment are spaced apart in the orthogonal direction, and, in the waistline conveyance step, the front waistline continuum and the rear waistline continuum are separately conveyed. Instead, the front waistline continuum and the rear waistline continuum may not be spaced apart in the orthogonal direction, and a waistline continuum in which the front waistline continuum and the rear waistline continuum are integrated may be used.

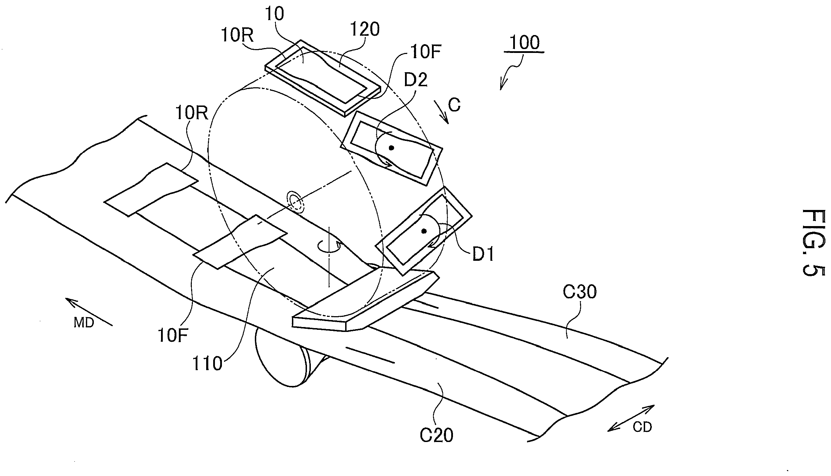

As illustrated in Fig. 3(b) and Fig. 5, in the main body arrangement step S5, the absorbent main bodies 10 are rotated and disposed on the waistline continuum. In the main body arrangement step S5, the absorbent main bodes 10 adjacent to each other in the conveyance direction MD, in a state of being cut in the cutting step S3, are rotated alternately in opposite directions. Specifically, one absorbent main body 10 is rotated by 90 degrees in a first direction D1 corresponding to a clockwise direction, and an absorbent main body 10 adjacent to the one absorbent main body 10 is rotated by 90 degrees in a second direction D2 corresponding to a counterclockwise direction. In a state after rotation, the front edges 10F of the absorbent main bodies 10 are arranged side by side along the conveyance direction MD, and the rear edges 10R of the absorbent main bodies 10 are arranged side by side along the conveyance direction MD. In the state after rotation, the width direction W of the absorbent main body 10 extends along the conveyance direction MD.

In the main body arrangement step S5, the absorbent main bodies 10 after rotation are disposed on the waistline continuum. In a configuration in which the front waistline continuum C20 and the rear waistline continuum C30 are spaced apart in the orthogonal direction CD as in the present embodiment, in the main body arrangement step S5, the absorbent main bodies 10 are disposed to straddle the front waistline continuum C20 and the rear waistline continuum C30. The front edges 10F of the absorbent main bodies 10 are disposed on the front waistline continuum C20 (a region of the waistline continuum on the front waistline region side), and the rear edges 10R of the absorbent main bodies 10 are disposed on the rear waistline continuum C30 (a region of the waistline continuum on the rear waistline region). In this way, it is possible to obtain a continuum in which the absorbent main bodies 10 are arranged in the width direction W, and the front waistline regions R2 and the rear waistline regions R3 are arranged in the width direction W, respectively.

In the main body arrangement step S5, the absorbent main bodies 10 are alternately rotated in opposite directions, so that the front edges 10F of the absorbent main bodies 10 can be disposed in the front waistline continuum C20 by rotation at an angle larger than 0 degree and smaller than 180 degrees. A rotation angle of the absorbent main body 10 may be set such that an angle obtained by adding a rotation angle of one absorbent main body 10 to a rotation angle of another absorbent main body 10 is 180 degrees. For example, the one absorbent main body 10 may be rotated clockwise by 80 degrees and the other absorbent main body may be rotated by 100 degrees.

In the main body arrangement step S5, the absorbent main bodies 10 adjacent to each other in the conveyance direction MD may be rotated by 90 degrees in opposite directions. When the absorbent main bodies 10 are alternately rotated by 90 degrees, angles of all the absorbent main bodies 10 are the same, and the absorbent main bodies 10 may be disposed in a certain direction. Since the absorbent main bodies 10 are rotated by 90 degrees at a time, rotation can be realized by a substantially common mechanism even in a reverse rotation. Addition to the mechanism due to acceleration etc. accompanying rotation corresponds to the same degree, and can be easily designed while suppressing defect of the mechanism.

In addition, in the main body arrangement step S5, positions in the orthogonal direction CD of the front edges 10F of the absorbent main bodies 10 may be aligned. When the positions of the front edges 10F of the absorbent main bodies 10 in the orthogonal direction CD coincide with each other, it is possible to continuously manufacture absorbent articles 1 in which positions of the absorbent main bodies 10 in the orthogonal direction CD are uniform. In addition, by causing positions of turning centers of a turning mechanism that turns the absorbent main bodies 10 to coincide with each other in the orthogonal direction, it is possible to suppress complication of a design of the turning mechanism when compared to a mechanism in which positions of turning centers are shifted in the orthogonal direction. In another embodiment, an adjustment mechanism for adjusting the positions of the absorbent main bodies in the orthogonal direction after rotation may be provided to align the positions of the front edges 10F of the absorbent main bodies 10 in the orthogonal direction CD.

For example, the main body arrangement step S5 can be implemented by a rotation mechanism illustrated in Fig. 5. Fig. 5 is a perspective view schematically illustrating a rotation mechanism 100 that implements the main body arrangement step S5. The rotation mechanism 100 has a rotating drum 110 and a plurality of pads 120 provided on an outer circumference of the rotating drum 110. The pads 120 receive the absorbent main bodies one by one, convey the absorbent main bodies 10 along a circumferential direction C with rotation of the rotating drums, and dispose the absorbent main bodies 10 on the front waistline continuum C20 and the rear waistline continuum C30. The pads 120 rotate by 90 degrees in a process of conveying the absorbent main bodies 10 along the circumferential direction C by a mechanism inside the rotating drum 110 to align directions of the absorbent main bodies 10. Pads 120 adjacent to each other in the circumferential direction C are independently rotatable and alternately rotate in opposite directions. By such a rotation mechanism, it is possible to alternately rotate absorbent main bodies 10 adjacent to each other in the conveyance direction MD in opposite directions and dispose the absorbent main bodies 10 in the waistline continuum in a state in which the directions of the absorbent main bodies 10 are aligned.

In the main body arrangement step S5, the absorbent main body 10 may be disposed on the waistline continuums C20 and C30 such that at least a part of the main body elastic members 14 overlaps the waistline elastic members 21 and 31. According to the absorbent article 1 in which at least a part of the main body elastic members 14 overlaps at least a part of the waistline elastic members 21 and 31, the main body elastic members 14 are pulled up by the waistline elastic members 21 and 31 at the time of wearing, and the absorbent main body 10 fits more to a body of the wearer.

In the extension and contraction suppression processing step S6, a process of suppressing a stretching force of the waistline elastic member is performed. Specifically, the waistline elastic member is cut or heat-welded to weaken the stretching force of the waistline elastic member or prevent the stretching force from developing. The extension and contraction suppression processing step S6 makes a processing pattern of the front waistline region and a processing pattern of the rear waistline region different from each other. Here, the processing pattern refers to an area subjected to extension and contraction suppression processing and a processing position. According to the absorbent article in which the processing pattern of the front waistline region is different from the processing pattern of the rear waistline region, an extension and contraction mode of the front waistline region is different from an extension and contraction mode of the rear waistline region, and it is possible to realize a shape or a fitting more suitable for the ventral portion and the gluteal portion. In addition, according to the method for manufacturing the absorbent article according to the present embodiment, since the pitch of the processing pattern corresponds to the length of one absorbent article, it is possible to suppress an increase in size of the production facility. Therefore, while suppressing an increase in size of the manufacturing facility, it is possible to make a processing pattern for suppressing extension and contraction of the waistline elastic member different between the front waistline region and the rear waistline region. The extension and contraction suppression processing step S6 is an arbitrary step, and may be performed as necessary.

Subsequently, as necessary, after the waistline continuum and the absorbent main body are folded in half along the conveyance direction using a folding line FL1 as a base point, and the outer side edge of the front waistline region and the outer side edge of the rear waistline region are cut, an individual absorbent article may be cut along the orthogonal direction, thereby obtaining the pants-type disposable diaper.

### INDUSTRIAL APPLICABILITY

According to a method for manufacturing the absorbent article that suppresses an increase in size of a production facility, it is possible to manufacture an absorbent article in which a structure of a front edge of an absorbent main body containing an absorber is different from a structure of a rear edge of the absorbent main body, and extension stress of a front waistline region is different from extension stress of a rear waistline region.

### REFERENCE SIGNS LIST

- 1: ABSORBENT ARTICLE
- 10: ABSORBENT MAIN BODY
- 10F: FRONT EDGE
- 10R: REAR EDGE
- 11: FRONT SHEET
- 12: ABSORBER
- 13: REAR SHEET
- 14: MAIN BODY ELASTIC MEMBER
- 20: FRONT WAISTLINE MEMBER (WAISTLINE MEMBER)
- 21: FRONT WAISTLINE ELASTIC MEMBER (WAISTLINE ELASTIC MEMBER)
- 30: REAR WAISTLINE MEMBER (WAISTLINE MEMBER)
- 31: REAR WAISTLINE ELASTIC MEMBER (WAISTLINE ELASTIC MEMBER)
- C10: MAIN BODY CONTINUUM
- C20: FRONT WAISTLINE CONTINUUM (WAISTLINE CONTINUUM)
- C30: REAR WAISTLINE CONTINUUM (WAISTLINE CONTINUUM)
- L: LONGITUDINAL DIRECTION
- W: WIDTH DIRECTION
- MD: CONVEYANCE DIRECTION
- CD: ORTHOGONAL DIRECTION
- S1: MAIN BODY CONVEYANCE STEP
- S2: ELASTIC MEMBER ARRANGEMENT STEP
- S3: CUTTING STEP
- S4: WAISTLINE CONVEYANCE STEP
- S5: MAIN BODY ARRANGEMENT STEP
- S6: EXTENSION AND CONTRACTION SUPPRESSION PROCESSING STEP

## Claims

1. A method for manufacturing an absorbent article (1),
the absorbent article (1) including an absorbent main body (10) that contains an absorber (12) and has a longitudinal direction (L) and a width direction (W) orthogonal to the longitudinal direction (L), and a waistline member (20, 30) that includes a waistline elastic member (21, 31) extending in the width direction (W) and that constitutes in a front waistline region (R2) and a rear waistline region (R3),
a structure of a front edge (10F) of the absorbent main body (10) being different from a structure of a rear edge (10R) of the absorbent main body (10), wherein a length of the rear edge (10R) of the absorbent main body (10) in the width direction (W) is longer than a length of the front edge (10F) of the absorbent main body (10) in the width direction (W), and
an extension stress of the front waistline region (R2) being different from an extension stress of the rear waistline region (R3),
the method comprising:
a main body conveyance step (S1) of conveying a main body continuum (C10) in which the absorbent main bodies (10) are continuous in the longitudinal direction (L), the front edges (10F) of the absorbent main bodies (10) are adjacent to each other, and the rear edges (10R) of the absorbent main bodies (10) are adjacent to each other;
a cutting step (S3) of cutting the main body continuum (C10) along the width direction (W) to obtain the individual absorbent main bodies (10);
a waistline conveyance step (S4) of conveying a waistline continuum (C20, C30) in which regions constituting the front waistline regions (R2) are continuous in a conveyance direction (MD), and regions constituting the rear waistline regions (R3) are continuous in the conveyance direction (MD); and
a main body arrangement step (S5) of rotating the absorbent main bodies (10) and disposing the absorbent main bodies (10) on the waistline continuum (C20, C30),
wherein in the main body arrangement step (S5), after absorbent main bodies (10) adjacent to each other in the conveyance direction (MD) are rotated in opposite directions, the front edges (10F) of the absorbent main bodies (10) are disposed in a region of the waistline continuum (C20) on a side of the front waistline regions (R2), and the rear edges (10R) of the absorbent main bodies (10) are disposed in a region of the waistline continuum (C30) on a side of the rear waistline regions (R3).

2. The method according to claim 1, wherein the main body arrangement step (S5) rotates the absorbent main bodies (10) adjacent to each other in the conveyance direction (MD) by 90 degrees in opposite directions.

3. The method according to claim 1 or 2, wherein the main body arrangement step (S5) aligns positions of the front edges (10F) of the absorbent main bodies (10) in an orthogonal direction (CD) orthogonal to the conveyance direction.

4. The method according to any one of claims 1 to 3, further comprising
an elastic member arrangement step (S2) of providing a main body elastic member (14) that extends and contracts in the longitudinal direction (L) on the main body continuum (C10) before the cutting step (S3),
wherein the elastic member arrangement step (S2) makes a position of a front edge (14F) of the main body elastic member (14) in the width direction (W) different from a position of a rear edge (14R) of the main body elastic member (14) in the width direction (W).

5. The method according to claim 4, wherein the main body arrangement step (S5) disposes the absorbent main body (10) on the waistline continuum (C20, C30) such that at least a part of the main body elastic member (14) overlaps the waistline elastic member (21, 31).

6. The method according to any one of claims 1 to 5,
wherein the waistline conveyance step (S4) separately conveys a front waistline continuum (C20) and a rear waistline continuum (C30) constituting the waistline continuum (C20, C30), and
the main body arrangement step (S5) disposes the absorbent main body (10) so that the absorbent main body (10) straddles the front waistline continuum (C20) and the rear waistline continuum (C30).

7. The method according to claim 6, wherein a structure of the front waistline continuum (C20) is different from a structure of the rear waistline continuum (C30).

8. The method according to any one of claims 1 to 7,
wherein the waistline elastic member (21, 31) is disposed in each of the front waistline region (R2) and the rear waistline region (R3),
the method further comprises an extension and contraction suppression processing step (S6) of suppressing a stretching force of the waistline elastic member (21, 31), and
a processing pattern of the front waistline region (R2) is different from a processing pattern of the rear waistline region (R3) in the extension and contraction suppression processing step (S6).

## Patentansprüche

1. Verfahren zur Herstellung eines absorbierenden Artikels (1), wobei der absorbierende Artikel (1) einen absorbierenden Hauptkörper (10), der einen Absorber (12) umfasst und eine Längsrichtung (L) und eine Breitenrichtung (W) orthogonal zu der Längsrichtung (L) aufweist, und ein Taillenelement (20, 30) umfasst, das ein elastisches Taillenelement (21, 31) umfasst, das sich in der Breitenrichtung (W) erstreckt und das einen vorderen Taillenbereich (R2) und einen hinteren Taillenbereich (R3) festlegt,
wobei eine Struktur einer Vorderkante (10F) des absorbierenden Hauptkörpers (10) sich von einer Struktur einer Hinterkante (10R) des absorbierenden Hauptkörpers (10) unterscheidet, wobei eine Länge der Hinterkante (10R) des absorbierenden Hauptkörpers (10) in der Breitenrichtung (W) länger ist als eine Länge der Vorderkante (10F) des absorbierenden Hauptkörpers (10) in der Breitenrichtung (W), und
wobei eine Dehnungsspannung des vorderen Taillenbereichs (R2) von einer Dehnungsspannung des hinteren Taillenbereichs (R3) verschieden ist,
wobei das Verfahren umfasst:
einen Hauptkörper-Förderschritt (S1) des Förderns eines Hauptkörperkontinuums (C10), in dem die absorbierenden Hauptkörper (10) in der Längsrichtung (L) kontinuierlich sind, wobei die Vorderkanten (10F) der absorbierenden Hauptkörper (10) aneinander angrenzen und die Hinterkanten (10R) der absorbierenden Hauptkörper (10) aneinander angrenzen;
einen Schneideschritt (S3) des Schneidens des Hauptkörperkontinuums (C10) entlang der Breitenrichtung (W), um die einzelnen absorbierenden Hauptkörper (10) zu erhalten;
einen Taillenförderschritt (S4) des Förderns eines Taillenkontinuums (C20, C30), in dem Bereiche, die die vorderen Taillenbereiche (R2) bilden, in einer Förderrichtung (MD) durchgehend sind, und Bereiche, die die hinteren Taillenbereiche (R3) bilden, in der Förderrichtung (MD) durchgehend sind; und
einen Hauptkörperanordnungsschritt (S5) des Rotierens der absorbierenden Hauptkörper (10) und des Anordnens der absorbierenden Hauptkörper (10) auf dem Taillenkontinuum (C20, C30),
wobei in dem Hauptkörperanordnungsschritt (S5), nachdem absorbierende Hauptkörper (10), die in der Förderrichtung (MD) aneinander angrenzen, in entgegengesetzte Richtungen gedreht werden,
wobei die Vorderkanten (10F) der absorbierenden Hauptkörper (10) in einem Bereich des Taillenkontinuums (C20) auf einer Seite der vorderen Taillenbereiche (R2) angeordnet sind, und die Hinterkanten (10R) der absorbierenden Hauptkörper (10) in einem Bereich des Taillenkontinuums (C30) auf einer Seite der hinteren Taillenbereiche (R3) angeordnet sind.

2. Verfahren nach Anspruch 1, wobei der Hauptkörperanordnungsschritt (S5) die aneinander angrenzenden absorbierenden Hauptkörper (10) in der Förderrichtung (MD) um 90 Grad in entgegengesetzte Richtungen rotiert.

3. Verfahren nach Anspruch 1 oder 2, wobei der Hauptkörperanordnungsschritt (S5) die Positionen der Vorderkanten (10F) der absorbierenden Hauptkörper (10) in einer orthogonalen Richtung (CD) orthogonal zur Förderrichtung ausrichtet.

4. Verfahren nach einem der Ansprüche 1 bis 3, ferner umfassend einen elastisches Element-Anordnungsschritt (S2) des Bereitstellens eines elastischen Hauptkörperelements (14), das sich in der Längsrichtung (L) auf dem Hauptkörperkontinuum (C10) vor dem Schneideschritt (S3) erstreckt und zusammenzieht, wobei der elastisches Element-Anordnungsschritt (S2) eine Position einer Vorderkante (14F) des elastischen Hauptkörperelements (14) in der Breitenrichtung (W) von einer Position einer Hinterkante (14R) des elastischen Hauptkörperelements (14) in der Breitenrichtung (W) verschieden macht.

5. Verfahren nach Anspruch 4, wobei der Hauptkörperanordnungsschritt (S5) den absorbierenden Hauptkörper (10) auf dem Taillenkontinuum (C20, C30) so anordnet, dass zumindest ein Teil des elastischen Hauptkörperelements (14) das elastische Taillenelement (21, 31) überlappt.

6. Verfahren nach einem der Ansprüche 1 bis 5,
wobei der Taillenförderschritt (S4) separat ein vorderes Taillenkontinuum (C20) und
ein hinteres Taillenkontinuum (C30) fördert, welche das Taillenkontinuum (C20, C30) ausbilden, und
der Hauptkörperanordnungsschritt (S5) den absorbierenden Hauptkörper (10) so anordnet, dass der absorbierende Hauptkörper (10) das vordere Taillenkontinuum (C20) und das hintere Taillenkontinuum (C30) überspannt.

7. Verfahren nach Anspruch 6, wobei sich die Struktur des vorderen Taillenkontinuums (C20) von der Struktur des hinteren Taillenkontinuums (C30) unterscheidet.

8. Verfahren nach einem der Ansprüche 1 bis 7,
wobei das elastische Taillenelement (21, 31) jeweils in dem vorderen Taillenbereich (R2) und dem hinteren Taillenbereich (R3) angeordnet ist,
wobei das Verfahren ferner einen Dehnungs- und Kontraktionsunterdrückungs-Verarbeitungsschritt (S6) zum Unterdrücken einer Dehnungskraft des elastischen Taillenelements (21, 31) umfasst, und
wobei ein Verarbeitungsmuster des vorderen Taillenbereichs (R2) sich von einem Verarbeitungsmuster des hinteren Taillenbereichs (R3) in dem Dehnungs- und Kontraktionsunterdrückungs-Verarbeitungsschritt (S6) unterscheidet.

## Revendications

1. Procédé de fabrication d'un article absorbant (1),
l'article absorbant (1) comprenant un corps principal absorbant (10) qui contient un absorbeur (12) et a une direction longitudinale (L) et une direction de la largeur (W) orthogonales à la direction longitudinale (L), et un élément de tour de taille (20, 30) qui comprend un élément de tour de taille élastique(21, 31) s'étendant dans la direction de la largeur (W) et qui constitue une région de tour de taille avant (R2) et une région de tour de taille arrière (R3),
une structure d'un bord avant (10F) du corps principal absorbant (10) étant différente d'une structure d'un bord arrière (10R) du corps principal absorbant (10), dans laquelle une longueur du bord arrière (10R) du corps principal absorbant (10) dans la direction de la largeur (W) est plus longue qu'une longueur du bord avant (10F) du corps principal absorbant (10) dans la direction de la largeur (W), et
une tension d'extension de la région de tour de taille avant (R2) étant différente d'une tension d'extension de la région de tour de taille arrière (R3),
le procédé comprenant :
une étape de transport de corps principal (S1) consistant à transporter un continuum de corps principal (C10) dans lequel les corps principaux absorbants (10) sont continus dans la direction longitudinale (L), les bords avant (10F) des corps principaux absorbants (10) sont adjacents les uns aux autres,
et les bords arrière (10R) des corps principaux absorbants (10) sont adjacents les uns aux autres ;
une étape de découpe (S3) consistant à découper le continuum de corps principal (C10) le long de la direction de la largeur (W) pour obtenir les corps principaux absorbants individuels (10) ;
une étape de transport de tour de taille (S4) consistant à transporter un continuum de tour de taille (C20, C30) dans lequel des régions constituant les régions de tour de taille avant (R2) sont continues dans une direction de transport (MD), et des régions constituant les régions de tour de taille arrière (R3) sont continues dans la direction de transport (MD) ; et
une étape d'agencement de corps principal (S5) consistant à faire tourner les corps principaux absorbants (10) et à disposer les corps principaux absorbants (10) sur le continuum de la tour de taille (C20, C30),
dans lequel, à l'étape d'agencement du corps principal (S5), après que les corps principaux absorbants (10) adjacents les uns aux autres dans la direction de transport (MD) ont été tournés dans des directions opposées, les bords avant (10F) des corps principaux absorbants (10) sont disposés dans une région du continuum de de tour de taille (G20) sur un côté des régions de tour de taille avant (R2), et les bords arrière (10R) des corps principaux absorbants (10) sont disposés dans une région du continuum de tour de taille (C30) sur un côté des régions de tour de taille arrière (R3).

2. Procédé selon la revendication 1, dans lequel l'étape d'agencement des corps principaux (S5) fait tourner les corps principaux absorbants (10) adjacents les uns aux autres dans la direction de transport (MD) de 90 degrés dans des directions opposées.

3. Procédé selon la revendication 1 ou 2, dans lequel l'étape d'agencement de corps principal (S5) aligne les positions des bords avant (10F) des corps principaux absorbants (10) dans une direction orthogonale (CD) qui est orthogonale à la direction de transport.

4. Procédé selon l'une quelconque des revendications 1 à 3, comprenant également
une étape d'agencement d'élément élastique (S2) consistant à fournir un élément élastique de corps principal (14) qui s'étend et se contracte dans la direction longitudinale (L) sur le continuum de corps principal (C10) avant l'étape de découpe (S3),
dans lequel l'étape d'agencement d'élément élastique (S2) forme une position d'un bord avant (14F) de l'élément élastique de corps principal (14) dans la direction de la largeur (W) différente d'une position d'un bord arrière (14R) de l'élément élastique de corps principal (14) dans la direction de la largeur (W).

5. Procédé selon la revendication 4, dans lequel l'étape d'agencement du corps principal (S5) dispose le corps principal absorbant (10) sur le continuum de la taille (020, C30) de sorte qu'au moins une partie de l'élément élastique du corps principal (14) chevauche l'élément élastique de la taille (21, 31).

6. Procédé selon l'une quelconque des revendications 1 à 5, dans lequel l'étape de transport de tour de taille (S4) transporte séparément un continuum de tour de taille avant (C20) et un continuum de tour de taille arrière (C30) constituant le continuum de tour de taille (C20, C30), et l'étape d'agencement du corps principal (S5) dispose le corps principal absorbant (10) de sorte que le corps principal absorbant (10) chevauche le continuum de taille avant (C20) et le continuum de taille arrière (C30).

7. Procédé selon la revendication 6, dans lequel une structure du continuum de taille avant (C20) est différente d'une structure du continuum de taille arrière (C30).

8. Procédé selon l'une quelconque des revendications 1 à 7,
dans lequel l'élément élastique de ceinture (21, 31) est disposé dans chacune parmi la région de ceinture avant (R2) et la région de ceinture arrière (R3),
le procédé comprend en outre une étape de traitement de suppression d'extension et de contraction (S6) consistant à supprimer une force d'étirement de l'élément élastique de ceinture (21, 31), et
un motif de traitement de la région de taille avant (R2) est différent d'un motif de traitement de la région de taille arrière (R3) dans l'étape de traitement de suppression d'extension et de contraction (S6).
